# EUROPÄISCHE PATENTSCHRIFT

(11) **EP 0 770 589 B1**
(45) Veröffentlichungstag und Bekanntmachung des Hinweises auf die Patenterteilung: **10.01.2001**
(21) Anmeldenummer: 96116284.9
(22) Anmeldetag: 11.10.1996
(51) Int. Cl.: C07C 37/20, C07C 39/17, C08G 85/00

(54) **Verfahren zur Herstellung von Bisphenolen unter Verwendung neuer Cokatalysatoren**
Process for the preparation of bisphenols using new co-catalysts
Procédé pour la préparation des bisphénols en utilisant des catalyseurs nouveaux

(30) Priorität: 24.10.1995 DE 19539444
(43) Veröffentlichungstag der Anmeldung: 02.05.1997
(73) Patentinhaber: BAYER AG, 51368 Leverkusen (DE)
(72) Erfinder: Wulff, Claus, Dr., 47800 Krefeld (DE); Fennhoff, Gerhard, Dr., 47877 Willich (DE); Eitel, Alfred, Dr., 41539 Dormagen (DE)

(56) Entgegenhaltungen:
- EP-A- 0 481 287
- DE-A- 3 619 450
- DE-A- 3 727 641
- DE-A- 3 832 396
- DE-A- 4 121 791

## Beschreibung

Die Erfindung betrifft die Synthese von Bisphenolen aus Monophenolen und Carbonylverbindungen wie Aldehyden und Ketonen mit konzentrierten Mineralsäuren wie Salzsäure und/oder Chlorwasserstoffgas als saure Katalysatoren und einem Mercaptan als Cokatalysator, der an eine im Reaktionsmedium unlösliche Matrix durch eine Ionenpaarbindung fixiert ist.

Die Kondensation von Phenolen und Carbonylverbindungen zur Bildung von Bisphenolen unter Verwendung von Katalysatoren wie Chlorwasserstoffsäure (US-A 31 82 308; 21 91 831) und auch schwefelhaltiger Verbindungen als Cokatalysatoren ist bekannt (z.B. aus US-A 24 68 982; 26 23 908 die Verwendung von Thioglykolsäure und 3-Mercaptopropionsäure, aus US-A 27 75 620 der Zusatz von Alkylmercaptanen, aus Chemical Abstracts 58, 1403e der Zusatz von Schwefelwasserstoff). Die bekannten schwefelhaltigen Cokatalysatoren können in der betrieblichen Praxis zu erheblichen Verfärbungen der Bisphenole und der aus ihnen hergestellten Folgeprodukte wie beispielsweise Polycarbonate, Copolycarbonate, Polyester, Copolyester und Epoxidharze führen, wenn sie nicht durch entsprechende Reinigungsmaßnahmen wie beispielsweise Kristallisation des Bisphenols und Auswaschen der Schwefelverbindungen rückstandsfrei abgetrennt werden. Insbesondere bei Bisphenolen, deren Synthesen sehr hohe Konzentrationen an Cokatalysatoren erfordern, ist das Abtrennen der schwefelhaltigen Komponenten oftmals schwierig und mit unerwünschten Verlusten an gewünschter Zielverbindung verbunden, insbesondere im Falle thermischer Belastung, z.B. bei der Aufarbeitung.

Demgegenüber lassen sich Cokatalysatoren leicht vollständig durch Filtrieren oder Zentrifugieren abtrennen, wenn sie nicht in homogener Phase zudosiert werden, sondern vor Zugabe zum Eduktgemisch bereits über ionische Bindungen an eine im Reaktionsmedium unlösliche Matrix gebunden sind. Diese Form der Cokatalysatoren gestattet es, extrem hohe Cokatalysatorkonzentrationen zu verwenden, ohne daß die oben geschilderten Qualitätsprobleme auftreten.

Die Synthese von Bisphenolen mit an eine Harzmatrix fixierten Cokatalysatoren ist in der DE-A 3 619 450 (z.B. mit Aminoalkylmercaptan-Einheiten) oder in der DE-A 3 727 641 (z.B. mit Thiazolidin-Einheiten) beschrieben, wobei die ebenfalls notwendige Katalysatorsäure in Form von Sulfonsäureresten auch an die Harzmatrix gebunden ist.

DE-A 38 32 396 offenbart die Herstellung von Bisphenolen unter Verwendung von Katalysatoren, wobei als Katalysatorsysteme entweder eine Kombination aus wäßriger Säure und Mercaptanen oder aber eine Kombination aus sulfonsauren Ionenaustauscherharzen und Mercaptanen eingesetzt werden.

DE-A 41 21 791 offenbart die Herstellung von Bisphenolen unter Einsatz von Katalysatoren. Es werden ausschließlich heterogene Katalysatoren verwendet, wobei sulfonsaure Ionenaustauscher, die gegebenenfalls modifiziert wurden, eingesetzt werden.

Diese Verfahren eignen sich jedoch nicht gleichermaßen für die Herstellung aller Bisphenole. Bisphenole aus Ketonen mit höheren Alkylgruppen wie Dihydroxydiphenylcycloalkane vom Typ des 1,1-Bis-(4-hydroxyphenyl)-3,3,5-trimethylcyclohexans lassen sich auf diese Weise nur mit vergleichsweise geringen Umsätzen des entsprechenden Ketons und geringer Selektivität hinsichtlich unerwünschter Nebenprodukte gewinnen.

Es wurde nun gefunden, daß Bisphenole der Formel (I) wie beispielsweise das 1,1-Bis-(4-hydroxyphenyl)-3,3,5-trimethylcyclohexan mit konzentrierten Mineralsäuren wie Salzsäure und/oder Chlorwasserstoffgas zusammen mit einem sulfonsauren Ionenaustauscherharz auf Basis einer vernetzten Polystyrolharzmatrix, dessen Sulfonsäuregruppen mit einem Aminomercaptan und/oder einem Thiazolidin teilweise, bevorzugt vollständig neutralisiert worden sind, problemlos mit hohen Ketonumsätzen und hohen Selektivitäten herstellen lassen, wobei der vollständige Ketonumsatz nach kürzeren Reaktionszeiten erreicht wird, als dies mit den entsprechenden in homogener Phase verwendeten Cokatalysatoren wie Mercaptanen der Fall ist.

Gegenstand der vorliegenden Erfindung ist somit ein Verfahren zur Herstellung von Bisphenolen der Formel (I) durch Umsetzung von Phenolen der Formel (V) und Ketonen der Formel (VI).

Bisphenole der Formel (I) 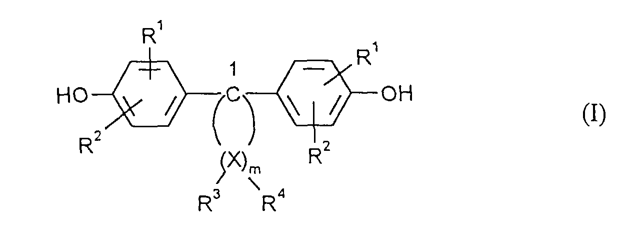 sind solche, worin
- R¹ und R²: unabhängig voneinander Wasserstoff, Halogen, bevorzugt Chlor oder Brom, C₁-C ₈-Alkyl, C₅-C₆-Cycloalkyl, C₆-C₁₀-Aryl, bevorzugt Phenyl, und C₇-C₁₂-Aralkyl, bevorzugt Phenyl-C₁-C₄-alkyl, insbesondere Benzyl,
- m: eine ganze Zahl von 4 bis 7, bevorzugt 4 oder 5,
- R³ und R⁴: für jedes X individuell wählbar, unabhängig voneinander Wasserstoff oder C₁-C₆-Alkyl und X
- X: Kohlenstoff bedeuten,
mit der Maßgabe, daß an mindestens einem Atom X R³ und R⁴ gleichzeitig Alkyl bedeuten; bevorzugt sind an 1 bis 2 Atomen X, insbesondere nur an einem Atom X, R³ und R⁴ gleichzeitig Alkyl.

Bevorzugter Alkylrest ist Methyl; die X-Atome in α-Stellung zu dem di-phenylsubstituierten C-Atom (C-1) sind bevorzugt nicht dialkylsubstituiert, dagegen ist die Alkyldisubstitution in β-Stellung zu C-1 bevorzugt. Insbesondere sind Gegenstand der Erfindung Dihydroxydiphenylcycloalkane mit 5 und 6 Ring-C-Atomen im cyclo-aliphatischen Rest (m = 4 oder 5) in Formel (I) wie beispielsweise die Diphenole der Formeln 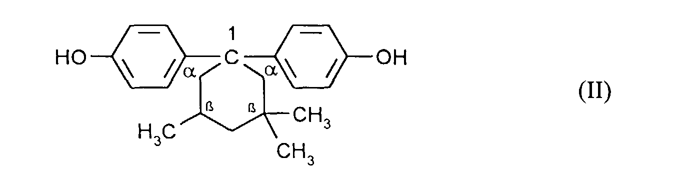 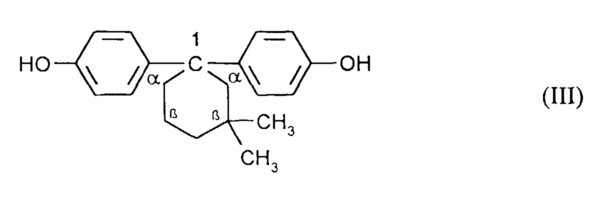 und 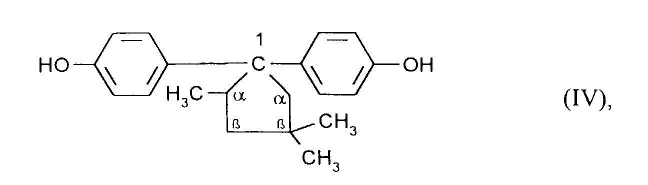 wobei das 1,1-Bis-(4-hydroxyphenyl)-3,3,5-trimethylcyclohexan (Formel (III)) besonders bevorzugt ist.

Phenole der Formel (V) 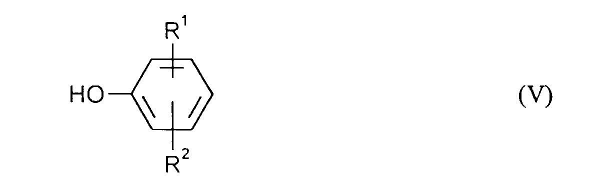 sind solche, worin R¹ die gleiche Bedeutung zukommt wie in Formel (I).

Die Phenole der Formel (V) sind entweder literaturbekannt oder nach literaturbekannten Verfahren erhältlich (siehe beispielsweise für Kresole und Xylenole, Ullmanns Encyclopädie der technischen Chemie, 4. neubearbeitete und erweiterte Auflage, Band 15, Seiten 61 bis 77, Verlag Chemie Weinheim-New York 1978; für Chlorphenole, Ullmanns Encyclopädie der technischen Chemie, 4. Auflage, Verlag Chemie, 1975, Band 9, Seiten 573 bis 582; und für Alkylphenole, Ullmanns Encyclopädie der technischen Chemie, 4. Auflage, Verlag Chemie 1979, Band 18, Seiten 191 bis 214).

Beispiele für geeignete Phenole der Formel (V) sind:
Phenol, o-Kresol, m-Kresol, 2,6-Dimethylphenol, 2-Chlorphenol, 3-Chlorphenol, 2,6-Dichlorphenol, 2-Cyclohexylphenol, Diphenylphenol und o- und p-Benzylphenole.

Ketone der Formel (VI) 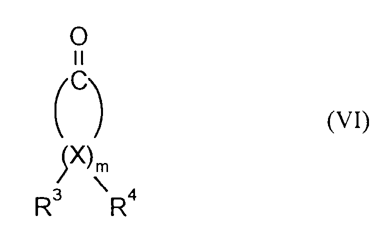 sind solche, worin X, R¹, R⁴ und m die gleichen Bedeutungen zukommen wie in Formel (I).

Beispiele für bekannte Ketone der Formel (VI) sind:
3,3-Dimethylcyclopentanon, 2,2-Dimethylcyclohexanon, 3,3-Dimethylcyclohexanon, 4,4-Dimethylcyclohexanon, 3-Ethyl-3-methylcyclopentanon, 2,3,3-Trimethylcyclopentanon, 2,4,4-Trimethylcyclopentanon, 3,3,4-Trimethylcyclopentanon, 3,3-Dimethylcycloheptanon, 4,4-Dimethylcycloheptanon, 3-Ethyl-3-4,4-Dimethylcyclohexanon, 4-Ethyl-4-methylcyclohexanon, 2,3,3-Trimethylcyclohexanon, 2,4,4-Trimethylcyclohexanon, 3,3,4-Trimethylcyclohexanon, 2,5,5-Trimethylcyclohexanon, 3,3,5-Trimethylcyclohexanon, 3,4,4-Trimethylcyclohexanon, 2,3,3,4-Tetramethylcyclopentanon, 2,3,4,4-Tetramethylcyclopentanon, 3,3,4,4-Tetramethylcyclopentanon, 2,2,5-Trimethylcycloheptanon, 2,2,6-Trimethylcycloheptanon, 2,6,6-Trimethylcycloheptanon, 3,3,5-Trimethylcycloheptanon, 3,5,5-Trimethylcycloheptanon, 5-Ethyl-2,5-dimethylcycloheptanon, 2,3,3,5-Tetramethylcycloheptanon, 2,3,5,5-Tetramethylcycloheptanon, 3,3,5,5-Tetramethylcycloheptanon, 4-Ethyl-2,3,4-trimethylcyclopentanon, 2-Isopropyl-4,4-dimethylcyclopentanon, 4-Isopropyl-2,4-dimethylcyclopentanon, 2-Ethyl-3,5,5-trimethylcyclohexanon, 3-Ethyl-3,5,5-trimethylcyclohexanon, 3-Ethyl-4-isopropyl-3-methyl-cyclopentanon, 4-sec,-Butyl-3,3-dimethylcyclopentanon, 2-Isopropyl-3,3,4-trimethylcyclopentanon, 3-Ethyl-4-isopropyl-3-methyl-cyclohexanon, 4-Ethyl-3-isopropyl-4-methyl-cyclohexanon, 3-sec.-Butyl-4,4-dimethylcyclohexanon, 3-Isopropyl-3,5,5-trimethylcyclohexanon, 4-Isopropyl-3,5,5-trimethylcyclohexanon, 3,3,5-Trimethyl-5-propylcyclohexanon, 3,5,5-Trimethyl-5-propylcyclohexanon, 2-Butyl-3,3,4-trimethylcyclopentanon, 2-Butyl-3,3,4-trimethylcyclohexanon, 4-Butyl-3,3,5-trimethylcyclohexanon, 3-Isohexyl-3-Isohexyl-3-methylcyclohexanon, 5-Ethyl-2,4-diisopropyl-5-methylcyclohexanon, 2,2-Dimethylcyclooctanon und 3,3,8-Trimethylcyclooctanon.

Beispiele für bevorzugte Ketone sind

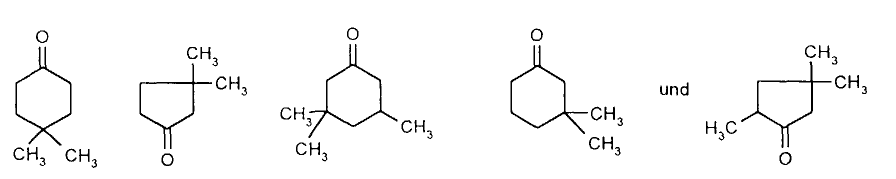

Die Ketone der Formel (VI) sind literaturbekannt (siehe beispielsweise) Beilsteins Handbuch der Organischen Chemie, 7. Band, 4. Auflage, Springer Verlag, Berlin, 1925, und die entsprechenden Ergänzungsbände 1 bis 4 und J. Am. Chem. Soc. Vol. 79 (1957), Seiten 1488, 1490 und 1491, US-PS 26 92 289, Allen et al., J. Chem. Soc., (1954), 2186, 2191 und J. Org. Chem. Vol. 38, No. 26 (1973), Seiten 4431 ff., J. Am. Chem. Soc. 87 (1965), Seite 1353 ff., insbesondere Seite 1355. Ein allgemeines Verfahren zur Herstellung von Ketonen der Formel (VI) ist beispielsweise in "Organikum", 15. Auflage, 1977, VEB-Deutscher Verlag der Wissenschaften, Berlin, beispielsweise Seite 698, beschrieben.

Das Verfahren ist dadurch gekennzeichnet, daß als Katalysator eine Mineralsäure, bevorzugt Salzsäure und/oder HCl-Gas zu einem Gemisch aus einem Phenol der Formel (V), einem Keton der Formel (VI) und einem sulfonsauren Ionenaustauscherharz auf Basis einer vernetzten Polystyrolharzmatrix, dessen Sulfonsäuregruppen mittels eines Mercaptoamins und/oder eines Thiazolidins zu mindestens 10 %, bevorzugt vollständig, neutralisiert worden sind, gegeben werden, wobei die Reaktionstemperaturen zwischen 20°C und 90°C, bevorzugt zwischen 25°C und 40°C gehalten werden. Die anschließende Abtrennung des sauren Ionenaustauscherharzes erfolgt durch Abfiltrieren bei 65°C bis 90°C, nachdem die Salzsäure und/oder das HCl-Gas abgetrennt worden ist, was vorzugsweise durch Verminderung des Druckes bei Temperaturen zwischen 30°C und 70°C erfolgt. Die Reinigung des Produktes erfolgt dann nach den üblichen Methoden durch Umkristallisieren in Phenol, Phenol und Wasser oder anderen geeigneten organischen Lösungsmitteln.

Als Mineralsäure wird bevorzugt eine Mischung von 36 %iger Salzsäure und HCl-Gas eingesetzt. Dabei werden je 18 bis 180 mol des Ketons der Formel (I) 1 kg 36 %ige Salzsäure und 1 kg HCl-Gas verwendet. Besonders bevorzugt wird HCl-Gas ohne Salzsäure eingesetzt, wobei in diesem Fall die Konzentration des HCl-Gases in der Reaktionsmischung zwischen 0,5 Gew.-% und 5,0 Gew.-%, bevorzugt zwischen 1,0 Gew.-% und 2,0 Gew.-%, bezogen auf das gesamte Reaktionsgemisch einschließlich des HCl-Gases liegt.

Als Cokatalysator wird ein sulfonsaures Ionenaustauscherharz auf Basis eines vernetzten Polystyrols eingesetzt, wobei die Säuregruppen des Ionenaustauscherharzes vor Einsetzen in der Reaktionsmischung durch Mercaptoamine und/oder Thiazolidine zu mindestens 10 %, bevorzugt jedoch vollständig neutralisiert worden sind und die Menge des eingesetzten erfindungsgemäßen Cokatalysators so gewählt wird, daß das molare Verhältnis von Mercaptogruppen zu Keton mindestens 1:10, bevorzugt 1:1 bis 10:1 beträgt.

Das saure Ionenaustauscherharz wird vor Verwendung in der Bisphenolsynthese solange mit destilliertem oder entsalztem Wasser gewaschen, bis das Waschwasser eine Leitfähigkeit von <100 µS aufweist. Anschließend wird durch Trocknung in herkömmlichen Trocknungsaggregaten oder durch Verdrängen mit wasserfreiem Phenol das Wasser aus dem Ionenaustauscherharz entfernt. Schließlich neutralisiert man in phenolischer Suspension das Ionenaustauscherharz mit einem Mercaptoamin und/oder einem Thiazolidin teilweise oder vollständig.

Der Vernetzungsgrad dieser Polystyrole kann zwischen 0,5 % und 50 %, vorzugsweise zwischen 1 % und 8 % liegen, wobei mit Vernetzungsgrad die eingesetzte Menge an vernetzend wirkendem Comonomer in Mol-% bezogen auf die eingesetzten Mole Styrol während der Copolymerisation gemeint ist.

Die Mercaptane sind bevorzugt ω-Aminoalkylmercaptane, als Thiazolidin eignet sich bevorzugt 2,2-Dimethylthiazolidin.

Die gemäß der vorliegenden Erfindung hergestellten Bisphenole eignen sich besonders für die Synthese von Polycarbonaten, Copolycarbonaten, Polyestern, Copolyestern und Epoxidharzen.

### Beispiele

### A. Vorbereitung des Ionentauscherharzes als Cokatalysator für die Bisphenolsynthese

300 g wasserfeuchtes, saures Ionenaustauscherharz (Lewatit SC 102, Bayer AG) werden solange mit vollentsalztem Wasser gewaschen, bis das Waschwasser eine Leitfähigkeit <100 µS aufweist. Anschließend saugt man das Lewatit über eine Saugnutsche scharf ab und trocknet es im Vakuumtrockenschrank 72 Stunden lang bei 100°C und 20 mbar. Die Auswaage nach Trocknung ergibt ca. 60 g trockenes Lewatit SC 102.

60 g trockenes Ionentauscherharz Lewatit SC 102 werden in 300 ml Phenol (Wassergehalt <0,1 %) 4 Stunden lang gerührt, damit es vollständig quellen kann. Anschließend gibt man 26,5 g 2,2-Dimethylthiazolidin in 100 ml Phenol hinzu und rührt bei 65°C 24 Stunden lang in einem Rundkolben. Danach wird das Harz abfiltriert, scharf abgesaugt und das gebildete Bisphenol A und nicht angebundenes Dimethylthiazolidin werden solange mit frischem Phenol herausgespült, bis im Eluat Bisphenol A und Dimethylthiazolidin nicht mehr nachgewiesen werden können.

### B. Synthese von TMC-BP

### Beisipiel 1 mit konz. Salzsäure und HCl-Gas:

In ein Gemisch aus 499 g Phenol, 64 g 3,3,5-Trimethylcyclohexanon, 3,74 g 36 %iger Salzsäure und 125 g des nach A. vorbereiteten Ionentauscherharzes wird in einer Rührapparatur bei 30°C 1 Stunde lang HCl-Gas eingeleitet. Man läßt noch 9 Stunden weiterrühren und bestimmt dann den Ketonumsatz und die Selektivität der Reaktion.
- Keton-Umsatz:: 97 bis 99 %
- Selektivität:: 85 % 1,1-Bis-(4-hydroxyphenyl)-3,3,5-trimethylcyclohexan

### Beispiel 2 nur mit HCl-Gas:

In ein Gemisch aus 499 g Phenol, 64 g 3,3,5-Trimethylcyclohexanon und 125 g des nach A. vorbereiteten Ionentauscherharzes wird in einer Rührapparatur bei 30°C 1 Stunde lang HCl-Gas eingeleitet. Man läßt noch 9 Stunden weiterrühren und bestimmt dann den Ketonumsatz und die Selektivität der Reaktion.
- Keton-Umsatz:: 97 bis 99 %
- Selektivität:: 90 % 1,1-Bis-(4-hydroxyphenyl)-3,3,5-trimethylcyclohexan

### Beispiel 3 (Vergleichsbeispiel entsprechend den herkömmlichen Herstellverfahren)

In ein Gemisch aus 499 g Phenol, 64 g 3,3,5-Trimethylcyclohexanon, 3,74 g 36 %iger Salzsäure und 0,19 g 3-Mercaptopropionsäuremethylester wird in einer Rührapparatur bei 30°C 1 Stunde lang HCl-Gas eingeleitet. Man läßt noch 19 Stunden weiterrühren und bestimmt dann den Ketonumsatz und die Selektivität der Reaktion.
- Keton-Umsatz nach 20 Stunden:: 100 %
- Selektivität:: 81 % 1,1-Bis-(4-hydroxyphenyl)-3,3,5-trimethylcyclohexan

## Patentansprüche

1. Verfahren zur Herstellung von Bisphenolen der Formel 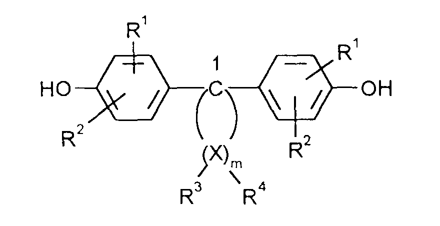 durch Umsetzung von Phenolen der Formel 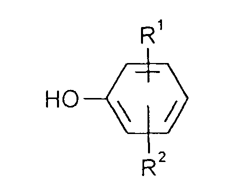 und Ketonen der Formel 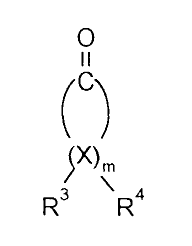 wobei
R¹ und R² unabhängig voneinander Wasserstoff, Halogen, C₁-C₈-Alkyl, C₅-C₆-Cycloalkyl, C₆-C₁₀-Aryl und C₇-C₁₂-Aralkyl,
m eine ganze Zahl von 4 bis 7,
R³ und R⁴ für jedes X individuell wählbar, unabhängig voneinander Wasserstoff oder C₁-C₆-Alkyl und
X Kohlenstoff bedeuten,
mit der Maßgabe, daß an mindestens einem Atom X R³ und R⁴ gleichzeitig Alkyl bedeuten,
dadurch gekennzeichnet, daß als Katalysator eine Mineralsäure verwendet und als Cokatalysator ein sulfonsaures Ionentauscherharz auf Basis eines vernetzten Polystyrols eingesetzt wird, wobei die Säuregruppen des Ionentauscherharzes vor Einsatz im Reaktionsgemisch durch Mercaptoamine und/oder Thiazolidine zu mindestens 10 % neutralisiert worden sind und die Menge des eingesetzten Cokatalysators so gewählt wird, daß das molare Verhältnis von Mercaptogruppen und eingesetztem Keton mindestens 1:10 beträgt.

2. Verfahren gemäß Anspruch 1, dadurch gekennzeichnet, daß das saure Ionenaustauscherharz vor Verwendung in der Bisphenolsynthese so lange mit destilliertem oder entsalztem Wasser gewaschen wird, bis das Waschwasser eine Leitfähigkeit von <100 µS aufweist, anschließend durch Trocknung in herkömmlichen Trocknungsaggregaten oder durch Verdrängen mit wasserfreiem Phenol das Wasser aus dem Ionenaustauscherharz entfernt wird und schließlich in phenolischer Suspension das Ionenaustauscherharz mit einem Mercaptoamin und/oder einem Thiazolidin teilweise oder vollständig neutralisiert wird.

3. Verfahren gemäß Anspruch 1, dadurch gekennzeichnet, daß das Ionenaustauscherharz nach Reaktionsende durch Abfiltrieren oder Zentrifugieren von den Reaktionsprodukten abgetrennt wird, nachdem die konzentrierte Säure und/oder das HCl-Gas aus dem Reaktionsgemisch entfernt worden sind.

## Claims

1. A process for the preparation of bisphenols of formula 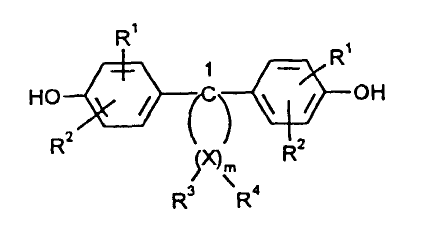 by the reaction of phenols of formula 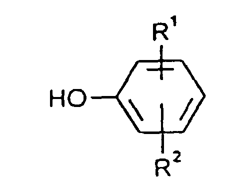 and ketones of formula 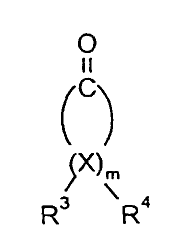 wherein
R¹ and R², independently of each other, denote hydrogen, a halogen, a C₁-C₈-alkyl, a C₅-C₆-cycloalkyl, a C₆-C₁₀-aryl or a C₇-C₁₂-aralkyl group,
m denotes an integer from 4 to 7,
R³ and R⁴ can be selected individually for each X, and independently of each other denote hydrogen or a C₁-C₆-alkyl group, and
X denotes carbon,
with the proviso that on at least one X atom R³ and R⁴ simultaneously denote an alkyl group,
characterised in that a mineral acid is used as a catalyst and a sulphonic acid ion-exchange resin based on a crosslinked polystyrene is used as a co-catalyst, wherein at least 10 % of the acid groups of the ion-exchange resin have been neutralised by mercaptoamines and/or thiazolidines before use in the reaction mixture, and the amount of co-catalyst used is selected so that the molar ratio of mercapto groups to the ketone used is at least 1:10.

2. A process according to claim 1, characterised in that before use in the bisphenol synthesis the acidic ion-exchange resin is washed with distilled or deionised water until the wash water has a conductivity of <100 µS, the water is subsequently removed from the ion-exchange resin by drying in conventional drying units or by displacement with anhydrous phenol, and the ion-exchange resin is finally partially or completely neutralised in phenolic suspension by a mercaptoamine and/or a thiazolidine.

3. A process according to claim 1, characterised in that after the completion of the reaction the ion-exchange resin is separated from the reaction products by filtration or centrifugation after the concentrated acid and/or HCl gas have been removed from the reaction mixture.

## Revendications

1. Procédé pour la préparation de bisphénols répondant à la formule 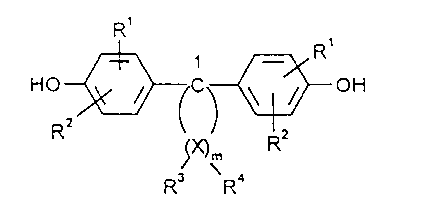 par mise en réaction de phénols répondant à la formule 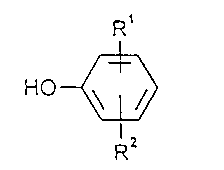 avec des cétones répondant à la formule 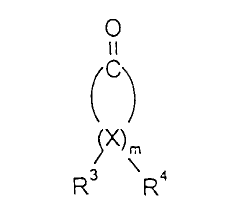 dans lesquelles
R¹ et R² représentent, indépendamment l'un de l'autre, un atome d'hydrogène, un atome d'halogène, un groupe alkyle en C₁-C₈, un groupe cycloalkyle en C₅-C₆, un groupe aryle en C₆-C₁₀ et un groupe aralkyle en C₇-C₁₂,
m représente un nombre entier de 4 à 7,
R³ et R⁴ représentent, qui peuvent faire l'objet d'une sélection individuelle pour chaque radical X, indépendamment l'un de l'autre, un atome d'hydrogène ou un groupe alkyle en C₁-C₆, et
X représente un atome de carbone,
avec cette mesure que, sur au moins un atome X, R³ et R⁴ représentent simultanément un groupe alkyle,
caractérisé en ce qu'on utilise, à titre de catalyseur, un acide minéral et, à titre de cocatalyseur, une résine échangeuse d'ions sulfonée acide à base d'un polystyrène réticulé, les groupes acides de la résine échangeuse d'ions ayant été neutralisés à concurrence d'au moins 10% en utilisant des mercaptoamines et/ou des thiazolidines avant la mise en ouvre dans le mélange réactionnel, et la quantité du cocatalyseur mis en ouvre étant sélectionnée de telle sorte que le rapport molaire des groupes mercapto et de la cétone mise en ouvre s'élève à au moins 1:10.

2. Procédé selon la revendication 1, caractérisé en ce qu'on lave la résine acide échangeuse d'ions avec de l'eau distillée ou dessalée avant son utilisation dans la synthèse du bisphénol jusqu'à ce que les eaux de lavage présentent une conductibilité < 100 µS, puis on élimine l'eau de la résine échangeuse d'ions par séchage dans des agrégats de séchage habituels ou par déplacement avec du phénol anhydre et enfin on neutralise partiellement ou complètement la résine échangeuse d'ions avec une mercaptoamine et/ou avec une thiazolidine dans une suspension phénolique.

3. Procédé selon la revendication 1, caractérisé en ce qu'on sépare la résine échangeuse d'ions des produits réactionnels après la fin de la réaction par filtration ou par centrifugation après avoir éliminé l'acide concentré et/ou le gaz chlorhydrique du mélange réactionnel.
